# EUROPEAN PATENT SPECIFICATION

(11) **EP 4 200 428 B1**
(45) Date of publication and mention of the grant of the patent: **19.02.2025**
(21) Application number: 21759123.9
(22) Date of filing: 20.08.2021
(51) Int. Cl.: C12N 15/86

(54) **METHOD OF MAKING RECOMBINANT AAVS**
VERFAHREN ZUR HERSTELLUNG VON REKOMBINANTEN AAVS
PROCÉDÉ DE FABRICATION D'AAV RECOMBINÉS

(30) Priority: 21.08.2020 GB 202013057
(43) Date of publication of application: 28.06.2023
(73) Proprietor: Oxford Genetics Limited, Oxford OX4 4HG (GB); Oxford University Innovation Limited, Oxford, OX2 0JB (GB)
(72) Inventor: CAWOOD, Ryan, Oxford, Oxfordshire OX4 4HG (GB); SU, Weiheng, Oxford, Oxfordshire OX4 4HG (GB)
(74) Representative: Dehns
(86) International application number: PCT/GB2021/052162
(87) International publication number: WO 2022/038369

(56) References cited:
- WO-A1-2019/020992
- WO-A2-03/104413
- PICCONI J L ET AL: "Kidney-specific expression of GFP by in-utero delivery of pseudotyped adeno-associated virus 9", MOLECULAR THERAPY- METHODS & CLINICAL DEVELOPMENT, vol. 1, 1 January 2014 (2014-01-01), GB, pages 14014, XP055765300, ISSN: 2329-0501, DOI: 10.1038/mtm.2014.14
- CONWAY J E ET AL: "High-titer recombinant adeno-associated virus production utilizing a recombinant herpes simplex virus type I vector expressing AAV-2 Rep and Cap", GENE THERAPY, vol. 6, no. 6, 1 June 1999 (1999-06-01), GB, pages 986 - 993, XP055731619, ISSN: 0969-7128, DOI: 10.1038/sj.gt.3300937

## Description

The present invention relates to a process for producing recombinant adeno-associated virus (AAV) particles, described herein as trans-pseudotyping. The process involves the production of recombinant AAV particles in first host cells and then in second host cells, wherein first and second AAV cap genes are expressed in the first and second host cells, respectively, thus producing first and second recombinant AAV particles which are encapsidated by first and second AAV capsid polypeptides. The first and second recombinant AAV particles have different cell tropisms, preferably towards production cell lines (for high efficiency production of AAVs) and for cells associated with a therapeutic indication (for treatment of such an indication), respectively.

Adeno-associated viruses (AAVs) are single-stranded DNA viruses that belong to the *Parvoviridae* family. It is a non-pathogenic virus that generates only a limited immune response in most patients. The virus cellular and tissue tropism is defined by the capsid on the surface of the AAV particles. Some capsids allow infection of a broad range of host cells, including both dividing and non-dividing cells, whilst others are considerably more restricted. Some do not infect standard production or manufacturing cell lines such as HEK293 cells.

Over the last few years, vectors derived from AAVs have emerged as an extremely useful and promising mode of gene delivery. This is owing to the following properties of these vectors:
- AAVs are small, non-enveloped viruses and they have only two native genes (rep and *cap*). Thus, they can be easily manipulated to develop vectors for different gene therapies. This is achieved by the removal of the rep and cap genes in the AAV genome and replacing these sequences with exogenous sequences (transgenes) that may provide therapeutic benefit to a patient.
- AAV particles are not easily degraded by shear forces, enzymes or solvents. This facilitates easy purification and final formulation of these viral vectors.
- AAVs are non-pathogenic and have a low immunogenicity. The use of these vectors further reduces the risk of adverse inflammatory reactions. Unlike other viral vectors, such as lentivirus, herpes virus and adenovirus, AAVs are harmless and are not thought to be responsible for causing any human disease.
- Genetic sequences up to approximately 4500 bp can be delivered into a patient using AAV vectors.
- Whilst wild-type AAV vectors have been shown to sometimes insert genetic material into human chromosome 19, this property is generally eliminated from most AAV gene therapy vectors by removing rep and cap genes from the viral genome. In such cases, the virus remains in an episomal form within the host cells. These episomes remain intact in non-dividing cells, while in dividing cells they are lost during cell division.

The native AAV genome comprises two genes each encoding multiple open reading frames (ORFs): the rep gene encodes non-structural proteins that are required for the AAV life-cycle and site-specific integration of the viral genome; and the cap gene encodes the structural capsid proteins. In addition, these two genes are flanked by inverted terminal repeat (ITR) sequences consisting of 145 bases that have the ability to form hairpin structures. These hairpin sequences are required for the primase-independent synthesis of a second DNA strand and the integration of the viral DNA into the host cell genome.

In order to eliminate any integrative capacity of the virus, recombinant AAV vectors remove *rep* and *cap* from the DNA of the viral genome. To produce such vectors, the desired transgene(s), together with a promoter(s) to drive transcription of the transgene(s), is inserted between the inverted terminal repeats (ITRs); and the *rep* and cap genes are provided in *trans* from either a second plasmid or a helper virus encoding the rep and or cap genes. Helper genes such as adenovirus E4, E2a and VA genes are also provided by either a plasmid or a helper virus. *rep, cap* and helper genes may be provided on additional plasmids that are transfected into cells or via a helper virus.

Traditionally, the production of AAV vectors has been achieved through a number of different routes.

Initially, AAV was generated using wild-type (WT) Adenovirus serotype 5 whilst transfecting cells with plasmids encoding the *rep* and *cap* genes and the AAV genome. This allowed the WT adenovirus to provide a number of factors in *trans* that facilitated virus replication. However, there are a number of limitations to this approach: for example, each batch of AAV must be separated from the Adenoviral (AV) particles after manufacture to provide a pure product and ensuring that all Ad5 has been removed is challenging. Moreover, the fact that during production the cell is devoting considerable resources to the production of Adenoviral particles rather than AAV is also undesirable.

In other systems, stable packing cell lines expressing the rep and cap genes have been used. In such systems, the *rep* and *cap* genes are integrated into the cell genomes, hence obviating the need for plasmid-based *rep* and *cap* genes. However, these genes are usually only integrated at low frequency (e.g. 1-2 copies per cell) due to their inherent toxicity. These systems require the infection with adenoviral vectors.

More recently, the adenovirus-based systems have been replaced with plasmids encoding the sections of the Adenovirus genome required for AAV production. Whilst this has solved some of the concerns over Adenovirus particles being present in the final virus preparation, a number of issues remain. These include the requirement to pre-manufacture sufficient plasmid for transfection into the production cell line and the inherently inefficient process of transfection itself.

In all current methods of producing AAVs, the AAV genome needs to be provided in *trans* via plasmid transfection, cell line integration, or via a helper virus, every time more AAV is to be created.

The inventors have now found that it is possible to propagate AAV particles containing an AAV genome that have already been produced by co-infecting cells with an adenovirus expressing the rep and cap genes and infectious AAV particles that have already been produced by one of the methods described above.

However, some AAV capsids used on the surface of AAV particles have selectivity for a target therapeutic tissue (e.g. retinal neurons or hepatocytes) and therefore this new approach to perpetually grow AAV only works if the AAV being produced is capable of infecting a production cell line (e.g. HEK293 cells). In many cases this is not possible or only occurs at low efficiency (e.g. such as for AAV6 and AAV9 in HEK293 cells).

The inventors describe herein a method to overcome this problem via the newly-termed process of trans-pseudotyping.

Each AAV particle will have a tropism towards a range of cells based on its capsid polypeptides, which are encoded from the cap gene. Some cap genes allow high efficiency infection of a production cell line (e.g. AAV2 capsid in HEK293 cells); other capsids do not allow high efficiency infection of a production cell line (e.g. AAV9 capsid in HEK293 cells). As described above, the inventors have developed a new way to produce AAVs by using AAVs to infect a production cell line and providing the *cap* gene in *trans.* Therefore, AAVs can initially be grown perpetually by providing a *cap* gene that endows the 'seed stock' AAV particles with an ability to infect a production cell line (e.g. AAV2 for HEK293 cells). Once enough seed-stock AAV material has been produced, coated in a capsid that infects a production cell line, a different capsid can be provided in *trans* in the final production step to produce AAV that has the desired new tropism. AAV particles produced bearing this alternative and second *cap* gene may not infect a production cell line and can then be used to infect cells associated with a therapeutic indication. The capsid on their surface may endow selectivity or exquisite specificity for infecting a target cell or tissue, but may not provide any, or reduced, infectivity for a production cell line. This process is called herein trans-pseudotyping.

Picconi *et al.* relates to kidney-specific expression of GFP by in utero delivery of pseudotyped adeno-associated virus 9 (Mol. Ther. Meth. Clin. Dev. vol. 1, 2014, page 14014). WO 03/104413 A2 discloses the production of pseudotyped recombinant AAV (rAAV) virions. Conway *et al.* discloses high-titer recombinant adeno-associated virus production utilizing a recombinant herpes simplex virus type I vector expressing AAV-2 Rep and Cap (Gene Ther. vol. 6, 1999, pages 986-993).

It is an object of the invention therefore to provide a process for producing recombinant AAV particles, wherein first recombinant AAV particles are produced in first host cells, the AAV particles having a tropism towards second host cells (e.g. a production cell line); and then culturing the second host cells under conditions such that second recombinant AAV particles are produced having a tropism to infect cells of a desired therapeutic target tissue or organ.

In one embodiment, the invention provides a process for producing recombinant adeno-associated virus (AAV) particles, the process comprising the steps:
(a) culturing a population of first host cells, each first host cell comprises:
   (i) a nucleic acid molecule encoding a recombinant AAV genome;
   (ii) a nucleic acid molecule encoding a first AAV *cap* gene which encodes first AAV capsid polypeptides, wherein the first AAV capsid polypeptides confer a tropism on AAV particles which comprise such polypeptides towards second host cells;
   (iii) a nucleic acid molecule encoding an AAV *rep* gene;
   (iv) nucleic acid molecules encoding viral helper genes;
   such that the expression of each of (i)-(iv) in the first host cells is sufficient to produce first recombinant AAV particles comprising the AAV genome in the first host cells, wherein the first AAV particles are encapsidated by first AAV capsid polypeptides;
(b) infecting a population of second host cells with the first recombinant AAV particles which are produced from Step (a);
(c) expressing in the population of second host cells:
   (i) a nucleic acid molecule encoding a second AAV *cap* gene which encodes second AAV capsid polypeptides, wherein the second AAV capsid polypeptides confer a tropism on AAV particles which comprise such polypeptides towards third host cells;
   (ii) a nucleic acid molecule encoding an AAV rep gene;
   (iii) nucleic acid molecules encoding viral helper genes;

   wherein all of (i)-(iii) are independently either present in the second host cells or are subsequently introduced into the second host cells,
   such that the expression of each of (i)-(iii) in the second cells is sufficient to produce second recombinant AAV particles comprising the recombinant AAV genome in the second host cells, wherein the second recombinant AAV particles are encapsidated by the second AAV capsid polypeptides;
(d) culturing the second host cells in a culture medium under conditions such that second recombinant AAV particles comprising the recombinant AAV genome are produced, the second recombinant AAV particles each being encapsidated by a capsid comprising the second capsid polypeptides;
   and optionally,
(e) purifying and/or isolating second recombinant AAV particles from the second host cells or from the culture medium.

### DETAILS OF SEQUENCES

The following sequences are given in the Sequence Listing, which forms part of the description of this patent application.

| **SEQ ID NO:** | **Description** | **Form** |
|---|---|---|
| 1 | AAV1 capsid | Nucleotide |
| 2 | AAV1 capsid | Amino acid |
| 3 | AAV2 capsid | Nucleotide |
| 4 | AAV2 capsid | Amino acid |
| 5 | AAV3 capsid | Nucleotide |
| 6 | AAV3 capsid | Amino acid |
| 7 | AAV4 capsid | Nucleotide |
| 8 | AAV4 capsid | Amino acid |
| 9 | AAV5 capsid | Nucleotide |
| 10 | AAV5 capsid | Amino acid |
| 11 | AAV6 capsid | Nucleotide |
| 12 | AAV6 capsid | Amino acid |
| 13 | AAV7 capsid | Nucleotide |
| 14 | AAV7 capsid | Amino acid |
| 15 | AAV8 capsid | Nucleotide |
| 16 | AAV8 capsid | Amino acid |
| 17 | AAV9 capsid | Nucleotide |
| 18 | AAV9 capsid | Amino acid |
| 19 | AAV2 *rep gene* | Nucleotide |
| 20 | AAV2 Rep78 | Nucleotide |
| 21 | AAV2 Rep78 | Amino acid |
| 22 | AAV2 Rep68 | Nucleotide |
| 23 | AAV2 Rep68 | Amino acid |
| 24 | AAV2 Rep52 | Nucleotide |
| 25 | AAV2 Rep52 | Amino acid |
| 26 | AAV2 Rep40 | Nucleotide |
| 27 | AAV2 Rep40 | Amino acid |
| 28 | TetR binding site | Nucleotide |
| 29 | Modified AV Major Late Promoter | Nucleotide |
| 30 | Modified AV Major Late Promoter | Nucleotide |

Adeno-associated viruses (AAV) are small (approx.20 nm) replication-defective, non-enveloped viruses. In some embodiments, the AAV is an Adeno-associated dependoparvovirus A. In other embodiments, the AAV is an Adeno-associated dependoparvovirus B.

AAV particles are formed from capsid proteins which encapsidate the ssDNA AAV genome. The wild-type AAV genome comprises two genes each encoding multiple open reading frames (ORFs): the rep gene encodes non-structural proteins that are required for the AAV life-cycle and site-specific integration of the viral genome; and the cap gene encodes the structural capsid proteins. As used herein, the term "recombinant AAV particle" refers to an AAV particle which comprises a recombinant AAV genome.

As used herein, the term "recombinant AAV genome" refers to an AAV genome comprising AAV inverted terminal repeats (ITRs) flanking an intervening sequence, preferably wherein the intervening sequence is more than 100bp. The intervening sequence does not comprise AAV *rep* or *cap* genes. Preferably, the intervening sequence comprises a transgene.

Preferably, the term "recombinant AAV genome" refers to an AAV genome comprising a transgene (in place of the *rep* and *cap* genes) flanked by AAV inverted terminal repeats (ITRs).

As used herein, the terms "AAV genome", "AAV Transfer vector" and "Transfer Plasmid" are used interchangeably herein. They all refer to a vector comprising 5'- and 3'-viral (preferably AAV) inverted terminal repeats (ITRs) flanking an intervening sequence.

The transgene may be a coding or non-coding sequence. It may be genomic DNA or cDNA. Preferably, the transgene encodes a polypeptide or a fragment thereof.

Preferably, the transgene is operably-associated with one or more transcriptional and/or translational control elements (e.g. an enhancer, promoter, terminator sequence, etc.).

In some embodiments, the transgene codes for a therapeutic polypeptide or a fragment thereof. Examples of preferred therapeutic polypeptides include antibodies, CAR-T molecules, scFV, BiTEs, DARPins and T-cell receptors. In some embodiments, the therapeutic polypeptide is a G-protein coupled receptor (GPCR), e.g. DRD1. In some embodiments, the therapeutic polypeptide is an immunotherapy target, e.g. CD19, CD40 or CD38. In some embodiments, the therapeutic polypeptide is a functioning copy of a gene involved in human vision or retinal function, e.g. RPE65 or REP.

In some embodiments, the therapeutic polypeptide is a functioning copy of a gene involved in human blood production or is a blood component, e.g. Factor IX, or those involved in beta and alpha thalassemia or sickle cell anaemia. In some embodiments, the therapeutic polypeptide is a functioning copy of a gene involved in immune function such as that in severe combined immune-deficiency (SCID) or Adenosine deaminase deficiency (ADA-SCID). In some embodiments, the therapeutic polypeptide is a protein which increases/decreases proliferation of cells, e.g. a growth factor receptor. In some embodiments, the therapeutic polypeptide is an ion channel polypeptide.

In some preferred embodiments, the therapeutic polypeptide is an immune checkpoint molecule. Preferably, the immune checkpoint molecule is a member of the tumour necrosis factor (TNF) receptor superfamily (e.g. CD27, CD40, OX40, GITR or CD137) or a member of the B7-CD28 superfamily (e.g. CD28, CTLA4 or ICOS). Preferably, the immune checkpoint molecule is PD1, PDL1, CTLA4, Lag1 or GITR. In some preferred embodiments, the transgene encodes a CRISPR enzyme (e.g. Cas9, dCas9, Cpf1 or a variant or derivative thereof) or a CRISPR sgRNA.

Step (a) comprises culturing a population of first host cells, each first host cell comprising (i) a nucleic acid molecule encoding a recombinant AAV genome.

The nucleic acid molecules referred to herein may be DNA or RNA, preferably DNA.

In one embodiment, the nucleic acid molecule may be in the form of a vector or plasmid comprising the nucleic acid molecule encoding a recombinant AAV genome.

In another embodiment, the nucleic acid molecule may be in the form of a recombinant AAV particle comprising the nucleic acid molecule encoding a recombinant AAV genome.

In this embodiment, the recombinant AAV particle is encapsidated by AAV capsid polypeptides which confer a tropism towards the first host cells.

The recombinant AAV particle may be made by any suitable method. General methods for producing recombinant AAV particles are well known in the art.

In another embodiment, the nucleic acid molecule may be in form of a recombinant adenovirus (AV) particle comprising the nucleic acid molecule encoding a recombinant AAV genome (e.g. such as that described in WO2019/020992 and further herein).

In another embodiment, the nucleic acid molecule encoding the recombinant AAV genome may be stably integrated into the genome of the first host cells.

In some embodiments of the invention, Step (a) additionally comprises the prior step of introducing a nucleic acid molecule encoding a recombinant AAV genome into the first host cells. The nucleic acid molecule encoding a recombinant AAV genome may be in any of the forms described above.

As used herein, the term "introducing" includes transformation, and any form of electroporation, conjugation, infection, transduction or transfection, *inter alia.* The term "introduced" is similarly interpreted, *mutatis mutandis.*

The first recombinant AAV particles comprise first AAV capsid polypeptides which confer a tropism towards the second host cells. The second recombinant AAV particles comprise second AAV capsid polypeptides which confer a tropism towards the third host cells.

AAV capsid polypeptides are encoded by the AAV *cap* gene. As used herein, the term "*cap* gene" refers to a gene that encodes one or more open reading frames (ORFs), wherein each of said ORFs encodes an AAV Cap structural protein, or variant or derivative thereof. These AAV Cap structural proteins (or variants or derivatives thereof) form the AAV capsid.

The three Cap proteins are VP1, VP2 and VP3, which are generally 87kDa, 72kDa and 62kDa in size, respectively. Hence the AAV *cap* gene is one which encodes the three Cap proteins VP1, VP2 and VP3. In the wild-type AAV, these three proteins are translated from the p40 promoter to form a single mRNA. After this mRNA is synthesized, either a long or a short intron can be excised, resulting in the formation of a 2.3 kb or a 2.6 kb mRNA. The AAV capsid is composed of 60 capsid protein subunits (VP1, VP2, and VP3) that are arranged in an icosahedral symmetry in a ratio of 1:1:10, with an estimated size of 3.9 MDa. As used herein, the term "*cap* gene" includes wild-type cap genes and derivatives thereof, and artificial *cap* genes which have equivalent functions.

The AAV cap gene sequences and Cap polypeptide sequences for AAV serotypes 1-9 are given herein in SEQ ID NOs: 1-18, respectively. As used herein, the term "cap gene" or Cap polypeptide-encoding sequence preferably includes, but is not limited to:
(a) a polynucleotide molecule whose nucleotide sequence comprises or consists of the nucleotide sequence given in any one of SEQ ID NOs: 1, 3, 5, 7, 9, 11, 13, 15 or 17 (preferably SEQ ID NO: 17);
(b) a polynucleotide molecule whose nucleotide sequence comprises or consists of a variant of the nucleotide sequence given in any one of SEQ ID NOs: 1, 3, 5, 7, 9, 11, 13, 15 or 17 (preferably SEQ ID NO: 17), the variant having at least 40%, 50%, 60%, 70%, 80%, 85%, 90%, 95% or 99% (preferably at least 95%) sequence identity to any one of SEQ ID NOs: 1, 3, 5, 7, 9, 11, 13, 15 or 17 (preferably SEQ ID NO: 17); and
(c) a polynucleotide molecule whose nucleotide sequence comprises or consists of a nucleotide sequence which encodes:
   (i) a polypeptide whose amino acid sequence is given in any one of SEQ ID NOs: 2, 4, 6, 8, 10, 12, 14, 16, or 18 (preferably SEQ ID NO: 18), or
   (ii) a variant of (i), the variant having at least 50%, 60%, 70%, 80%, 85%, 90%, 95% or 99% (preferably at least 95%) sequence identity to any one of SEQ ID NOs: 2, 4, 6, 8, 10, 12, 14, 16, or 18 (preferably SEQ ID NO: 18).

Preferably, the variant is or encodes one or more VP1, VP2 and VP3 polypeptides. The first recombinant AAV particles are encapsidated by capsids comprising first AAV capsid polypeptides which confer a tropism towards the second host cells, and optionally also the first host cells. The second host cells are therefore ones which are capable of being infected by AAV particles which are encapsidated by first AAV capsid polypeptides.

The second recombinant AAV particles are encapsidated by capsids comprising second AAV capsid polypeptides which confer a tropism towards the third host cells (target cells). The third host cells (target cells) are therefore ones which are capable of being infected by AAV particles which are encapsidated by second AAV capsid polypeptides.

Preferably, the second AAV capsid polypeptides do not confer a tropism towards the second host cells, i.e. the second host cells are not capable of being infected at high efficiency with the second recombinant AAV particles. In this regard, the term "high efficiency" may be defined as requiring more than 100 viral particles per cell to obtain detectable transgene expression.

The first and second AAV capsid polypeptides will therefore have different amino acid sequences. Preferably, the amino acid sequence identity between the first and second AAV capsid polypeptides is less than 99.5%, e.g. less than 99%, 98%, 97%, 96%, 95% or less than 90%, and at least 50%. Preferably, the amino acid sequence identity between the first and second AAV capsid polypeptides is between 80% and 95%. Preferably, the amino acid sequences of the first and second AAV capsid polypeptides are different in at least 1, 2, 3, 4, 5, 6, 7, 8, 9, 10 or more amino acids, e.g. 1-5, 5-10 or 10-20 amino acids.

The first AAV cap gene is not expressed in the second host cells. The second AAV cap gene is not expressed in the first host cells.

AAV serotypes are determined by the AAV host cell tropisms. A serotype is a distinct variation within a species of virus. These viruses are classified together based on their cell surface (i.e. capsid) antigens, allowing the epidemiologic classification of viruses to the subspecies level.

AAV capsid proteins contain 12 hypervariable surface regions. The naturally-occurring capsid polypeptides vary from each other in their amino acid sequences. The primary regions of variation are recognised as being within the VP3 region of the capsid genes. However, the VP3 region is shared with the VP1 and VP2 coding sequences. Therefore, it is more accurate to say that the variable regions of the Cap polypeptides are more commonly found in the C-terminal half of the *cap* gene and, as such, the variation is generally shared by all of the capsid coding sequences. Preferably, therefore, the first and second recombinant AAV particles are of different serotypes.

11 different AAV serotypes are known. All of the known serotypes can infect cells from multiple diverse host cell types. The serotypes of the first and second recombinant AAV particles may be selected from the group consisting of serotypes 1, 2, 3, 4, 5, 6, 7, 8, 9, 10 and 11, or modified or mutated versions of these serotypes. Preferably, the serotypes of the first and second recombinant AAV particles are selected from the group consisting of serotypes 1, 2, 5, 6, 7, 8 or 9, or modified or mutated versions of these serotypes.

Most preferably, the first recombinant AAV particles are of serotype 2 (i.e. AAV2). Most preferably, the second recombinant AAV particles are of serotype 9 (i.e. AAV9).

Even more preferably, the second recombinant AAV particles are of a serotype, or modified or mutated derivative of a serotype, that has an increased tropism for the third host cells (target cells) compared to the tropism of AAV1-9.

As used herein, the term "rep gene" refers to a gene that encodes one or more open reading frames (ORFs), wherein each of said ORFs encodes an AAV Rep non-structural protein, or variant or derivative thereof. These AAV Rep non-structural proteins (or variants or derivatives thereof) are involved in AAV genome replication and/or AAV genome packaging.

The wild-type rep gene comprises three promoters: p5, p19 and p40.

Two overlapping messenger ribonucleic acids (mRNAs) of different lengths can be produced from p5 and from p19. Each of these mRNAs contains an intron which can be either spliced out or not using a single splice donor site and two different splice acceptor sites. Thus, six different mRNAs can be formed, of which only four are functional. The two mRNAs that fail to remove the intron (one transcribed from p5 and one from p19) read through to a shared terminator sequence and encode Rep78 and Rep52, respectively. Removal of the intron and use of the 5'-most splice acceptor site does not result in production of any functional Rep protein - it cannot produce the correct Rep68 or Rep40 proteins as the frame of the remainder of the sequence is shifted, and it will also not produce the correct C-terminus of Rep78 or Rep52 because their terminator is spliced out. Conversely, removal of the intron and use of the 3' splice acceptor will include the correct C-terminus for Rep68 and Rep40, whilst splicing out the terminator of Rep78 and Rep52. Hence the only functional splicing either avoids splicing out the intron altogether (producing Rep78 and Rep52) or uses the 3' splice acceptor (to produce Rep68 and Rep40). Consequently, four different functional Rep proteins with overlapping sequences can be synthesized from these promoters.

In the wild-type *rep* gene, the p40 promoter is located at the 3' end. Transcription of the Cap proteins (VP1, VP2 and VP3) is initiated from this promoter in the wild-type AAV genome.

The four wild-type Rep proteins are Rep78, Rep68, Rep52 and Rep40. Hence the wild-type rep gene is one which encodes the four Rep proteins Rep78, Rep68, Rep52 and Rep40.

As used herein, the term "*rep* gene" includes wild-type *rep* genes and derivatives thereof; and artificial *rep* genes which have equivalent functions. The wild-type *rep* gene encodes Rep78, Rep68, Rep52 and Rep40 polypeptides.

The full wild-type AAV (serotype 2) *rep* gene nucleotide sequence is given in SEQ ID NO: 19. The wild-type AAV (serotype 2) Rep78, Rep68, Rep52 and Rep40 nucleotide sequences are given herein in SEQ ID NOs: 20, 22, 24 and 26, respectively. The wild-type AAV (serotype 2) Rep78, Rep68, Rep52 and Rep40 amino sequences are given herein in SEQ ID NOs: 21, 23, 25 and 27, respectively.

As used herein, the term "*rep* gene" or Rep polypeptide-encoding sequence preferably includes, but is not limited to:
(a) a polynucleotide molecule whose nucleotide sequence comprises or consists of the nucleotide sequence given in any one of SEQ ID NOs: 19, 20, 22, 24 or 26 (preferably, SEQ ID NO: 19); and
(b) a polynucleotide molecule whose nucleotide sequence comprises or consists of a variant of the nucleotide sequence given in any one of SEQ ID NOs: 19, 20, 22, 24 or 26 (preferably SEQ ID NO: 19), the variant having at least 80%, 85%, 90%, 95% or 99% (preferably at least 95%) sequence identity to any one of SEQ ID NOs: 19, 20, 22, 24, or 26 (preferably SEQ ID NO: 19); and
(c) a polynucleotide molecule whose nucleotide sequence comprises or consists of a nucleotide sequence which encodes:
   (i) a polypeptide whose amino acid sequence is given in any one of SEQ ID NOs: 21, 23, 25 or 27, (preferably SEQ ID NO: 21), or
   (ii) a variant of (i), the variant having at least 80%, 85%, 90%, 95% or 99% (preferably at least 95%) sequence identity to any one of SEQ ID NOs: 21, 23, 25 or 27 (preferably SEQ ID NO: 21).

Preferably, the variant is or encodes one or more Rep78, Rep68, Rep52 and Rep40 polypeptides.

The rep and cap genes (and each of the protein-encoding ORFs therein) may be from one or more different viruses (e.g. 2, 3 or 4 different viruses). For example, the *rep* gene may be from AAV2, whilst the *cap* gene may be from AAV5.

It is recognised by those in the art that the *rep* and *cap* genes of AAV vary by clade and isolate. The sequences of these genes from all such clades and isolates are encompassed herein, as well as derivatives thereof.

Nucleic acid molecules encoding viral helper genes are also expressed in the host cells. Helper genes are required to aid the replication of the AAV genome and the production of infectious AAV particles. Suitable viral helper genes include one or more or all of E1A, E1B, E4, and VA RNA, and optionally an E2A gene. The helper genes are viral helper genes, preferably from adenovirus, herpesvirus or poxvirus. Most preferably, the helper genes are adenovirus helper genes. Some host cells (e.g. HEK293 cells) have the E1A and E1B genes stably integrated into their genomes and hence it is not necessary to introduce further copies of these latter genes into such host cells.

In order to produce recombinant AAV particles comprising the recombinant AAV genome, the AAV genome must be present in the host cells, and Rep and Cap polypeptides must be produced within the host cells or provided *in trans.* Additionally, sufficient viral helper genes (e.g. adenovirus E4, E1A, E1B and VA RNA, and optionally an E2A gene) are needed.

As used herein, the term "introducing" includes transformation, and any form of electroporation, conjugation, infection, transduction or transfection, *inter alia.* The term "introduced" is similarly interpreted, *mutatis mutandis.*

The nucleic acid molecules encoding the AAV cap and rep genes and the viral helper genes may independently be present in the first and/or second host cells or independently introduced into the first and/or second host cells in one or more of the following forms:
(i) stably integrated into the host cell's genome;
(ii) present episomally within the host cell;
(iii) present in a recombinant adenovirus in the host cell;
(iv) introduced into the host cell in a recombinant adenovirus; or
(v) introduced into the host cell in a vector or plasmid.

The forms of the nucleic acid molecules encoding the AAV cap and rep genes and the viral helper genes in the first and second host cells may be the same or different.

In Step (a), in embodiments wherein the above (iv) or (v) are introduced into the host cells, this introduction may be before or after introduction of the nucleic acid encoding a recombinant AAV genome.

Preferably, independently for the first and second host cells, the nucleic acid molecule encoding the AAV cap gene is in the form of a vector or a plasmid, or in a recombinant adenovirus.

Preferably, the vector, plasmid or recombinant adenovirus is introduced into the host cell, either before or after introduction of the nucleic acid encoding a recombinant AAV genome or transfection with the recombinant AAV particle.

Most preferably, the nucleic acid molecule encoding the first AAV cap gene is introduced into the first host cells in the form of a plasmid or in an AV recombinant genome (e.g. such as that described in WO2019/020992 and further herein).

Most preferably, the nucleic acid molecule encoding the second AAV cap gene is introduced into the second host cells in an AV recombinant genome (e.g. such as that described in WO2019/020992 and further herein).

Preferably, the nucleic acid molecule encoding the AAV rep gene is introduced into the first host cells in the form of a plasmid or in an AV recombinant genome (e.g. such as that described in WO2019/020992 and further herein). Preferably, the nucleic acid molecule encoding the AAV rep gene is introduced into the second host cells in an AV recombinant genome (e.g. such as that described in WO2019/020992 and further herein).

Preferably, the nucleic acid molecules encoding the viral helper genes are in the form of a helper adenovirus. Preferably, AV E1a and E1b genes are integrated into the host cell genome (e.g. as in HEK293 cells).

Most preferably, components (i)-(iv) of Step (a) will be delivered simultaneously. As mentioned above, the AAV *rep* gene and/or the AAV cap gene may independently be introduced (e.g. transfected) into the host cell in one or more recombinant adenoviruses or be present in one or more recombinant adenoviruses which are already in the host cell.

In order to accommodate the AAV *rep* gene and/or the AAV *cap* gene, part or all of one or more adenoviral genes may be deleted. These may include helper genes such as E1a and E1b that are integrated into the host cell genome (e.g. in HEK293 cells)

For example, the AAV *rep* gene and/or the AAV *cap* gene may be inserted into one of the adenoviral Early genes or inserted in a site from which Early genes have been deleted from an adenovirus. In the latter example, the deleted Early genes may be trans-complimented by a cell line containing the deleted genes, e.g. HEK293 cells which contain the adenoviral E1A and E1B regions.

The AAV *rep* gene and/or the AAV *cap* gene may be inserted into a region of an adenoviral genome containing an E1 deletion. In other instances, genes that are non-essential to the adenovirus can also be deleted and these sites can be used to insert a nucleic acid molecule of the invention. For example, the AAV *rep* gene and/or the AAV *cap* gene may be inserted in the E3 region of an adenovirus because most E3 genes can be deleted in an adenoviral vector.

The AAV *rep* gene and/or the AAV *cap* gene may be inserted into an adenoviral gene in sense or antisense orientation (with respect to the direction of transcription of the adenoviral gene). It is a preferred embodiment of the invention that the AAV *rep* gene and/or the AAV *cap* gene will be in the same direction of transcription as the E4, E2A and E2B expression cassettes when it is inserted into the E1 region. This is to prevent the E1A promoter (that is often retained in E1-deleted AV's) from acting as a promoter to drive the *rep* gene expression. The E1A promoter cannot be removed because it contains the AV packaging signal.

It is a preferred embodiment of the invention that the Rep-coding sequence will not contain an upstream promoter.

In some preferred embodiments, the AAV *rep* gene and/or the AAV i gene is inserted into an adenoviral E1 gene, preferably wherein part of the E1 gene has been deleted. Preferably, the E1 gene is E1A and/or E1B.

The *rep* and *cap* genes and the viral helper genes may each independently be operably-associated with a promoter, e.g. a constitutive promoter, an inducible promoter, a repressible promoter, a minimal promoter, or with no promoter. As used herein, the term "operably-associated" in the context of a promoter and a gene means that the promoter and the gene in question are located within a distance from each other which is sufficiently close for the promoter to promote transcription of the gene. In some embodiments, the promoter and the gene are juxtaposed or are contiguous.

Preferably, the promoters which are operably-associated with the AAV *cap* gene are constitutive or inducible promoters, more preferably constitutive promoters such as a minimal CMV promoter. In some embodiments, the first and/or second AAV *cap* gene is operably-associated with no promoter or with a minimal promoter.

Preferably, when the promoter which is operably-associated with the AAV cap gene is encoded within a recombinant AV, the promoter used will be selected based on the toxicity of the *cap* gene to the adenovirus and the expression levels required. The inventors have found that some *cap* genes can be expressed under the CMV promoter and have little to no impact on AV replication, e.g. AAV9. Conversely, some *cap* genes can only be inserted into AVs if driven by a minimal CMV promoter that has comparatively low expression, e.g. AAV6. Therefore, the promoter driving the *cap* gene will preferably be based on experimentally testing a low- and high-expressing promoter, where the high-expressing promoter is the CMV promoter and the low expression is the minimal promoter region from the same promoter.

In some embodiments, the Rep polypeptide is expressed at a low, baseline or minimal level. In some embodiments, the AAV *rep* gene is not operably-associated with any functional promoter. As used herein, the term "low, baseline or minimal level" refers to a level of expression of the Rep78 polypeptide which is less than 50%, 40%, 30%, 20% or 10% of the level of expression of a wild-type Rep 78 polypeptide which is operably-associated with a wild-type p5 promoter (in a wild-type AAV *rep* gene). In this way, sufficient Rep polypeptide is provided in order to enable the production of at least some AAV, but the level of Rep polypeptide expression is insufficient to completely inhibit adenovirus replication.

Preferably, the promoters which are operably-associated with the helper genes are constitutive or inducible promoters, more preferably constitutive promoters.

Examples of constitutive promoters include the CMV, SV40, PGK (human or mouse), HSV TK, SFFV, Ubiquitin, Elongation Factor Alpha, CHEF-1, FerH, Grp78, RSV, Adenovirus E1A, CAG or CMV-Beta-Globin promoter, or a promoter derived therefrom.

Preferably, the rep gene promoter is the SV40 promoter, or a promoter which is derived therefrom, or a promoter of equal or decreased strength compared to the SV40 promoter in human cells and human cell lines (e.g. HEK-293 cells).

In some embodiments, the promoter is inducible or repressible by the inclusion of an inducible or repressible regulatory (promoter) element. For example, the promoter may be one which is inducible with doxycycline, tetracycline, IPTG or lactose.

The *rep* genes and the cap genes may each also independently be operably-associated with a terminator, e.g. an SV40 polyadenylation signal.

In embodiments of the invention wherein the nucleic acid molecule encoding the recombinant AAV genome is in the form of a recombinant AAV particle, the recombinant AAV particle is encapsidated by AAV capsid polypeptides which confer a tropism towards the first host cells. The second host cells are ones which are capable of being infected by AAV particles which are encapsidated by first AAV capsid polypeptides. The third host cells (target cells) are ones which are capable of being infected by AAV particles which are encapsidated by second AAV capsid polypeptides.

The aim of Step (a) is to produce a quantity of recombinant AAV particles for use in Step (b). Hence the first host cells are preferably ones which are capable of producing high titres of first recombinant AAV particles. In some embodiments, the first host cells are from an AAV production cell line or an AAV manufacturing cell line, i.e. a cell line which comprises all of the polypeptides which are necessary for AAV capsid production and AAV maturation. The first host cells may comprise one or more different types of cells (e.g. a mixture of HEK293 and PerC6 cells). Preferably, the first host cells are all of the same type.

The first host cell is preferably a mammalian cell. Examples of mammalian cells include those from any organ or tissue from humans, mice, rats, hamsters, monkeys, rabbits, donkeys, horses, sheep, cows and apes. Preferably, the cells are human cells. The cells may be primary or immortalised cells.

Preferred first host cells include HEK293, HEK293T, HEK-293E, HEK-293 FT, HEK-293S, HEK-293SG, HEK-293 FTM, HEK-293SGGD, HEK-293A, MDCK, C127, A549, HeLa, CHO, mouse myeloma, PerC6, 911 and Vero cell lines. HEK-293 cells have been modified to contain the E1A and E1B proteins and this obviates the need for these proteins to be supplied on a Helper Plasmid. Similarly, PerC6 and 911 cells contain a similar modification and can also be used. Most preferably, the human cells are HEK293, HEK293T, HEK293A, PerC6, 911 or HeLaRC32. Other preferred cells include Hela, CHO and VERO cells. Most preferably, the first host cell is a HEK293, HEK293T, HEK293A, PerC6 or 911 cell.

The aim of Step (a) is to produce a quantity of first recombinant AAV particles for use in Step (b). Step (b) comprises infecting a population of second host cells with the first recombinant AAV particles which are produced from Step (a).

In this step, recombinant AAV particles are contacted with a population of second host cells in order to infect those second host cells with the first recombinant AAV particles. These second host cells will be capable of being infected with the first recombinant AAV particles because the AAV capsids which encapsidate the first recombinant AAV particles confer a tropism towards the second host cells.

Preferably, the infecting step in Step (b) is carried out using a composition comprising purified AAV particles, i.e. wherein the composition does not comprise any first host cells, preferably wherein the composition does not comprise any cells.

The aim of Step (d) is to produce a quantity of recombinant AAV particles for subsequent therapeutic use. Hence the second host cells are preferably ones which are capable of producing high titres of second recombinant AAV particles.

The second host cells may be any of the types of first host cells, or mixtures thereof. Most preferably, the second host cell is a HEK293, HEK293T, HEK293A, PerC6 or 911 cell.

The types of first host cells may be the same or different from the types of second host cells.

Preferred combinations of first/second host cells and AAV particles of a particular serotype which have a tropism for those host cells are given in the table below:

| **First host cell or second host cell** | **AAV serotype of first recombinant AAV particle** |
|---|---|
| HEK293 or a derivative thereof | AAV1, AAV2, AAV3, AAV6 |
| PerC6 or a derivative thereof | AAV2 |
| 911 or a derivative thereof | AAV2 |
| HeLa or a derivative thereof | AAV1, AAV2, AAV3, AAV6 |
| A549 or a derivative thereof | AAV2 |

The first and/or second host cells may be recombinant host cells.

Step (c) comprises:
(c) expressing in the population of second host cells:
(i) a nucleic acid molecule encoding a second AAV cap gene which encodes second AAV capsid polypeptides, wherein the second AAV capsid polypeptides confer a tropism on AAV particles which comprise such polypeptides towards third host cells;
(ii) a nucleic acid molecule encoding an AAV rep gene;
(iii) nucleic acid molecules encoding viral helper genes;

wherein all of (i)-(iii) are independently either present in the second host cells or are subsequently introduced into the second host cells,
such that the expression of each of (i)-(iii) in the second cells is sufficient to produce second recombinant AAV particles comprising the recombinant AAV genome in the second host cells,
wherein the second recombinant AAV particles are encapsidated by the second AAV capsid polypeptides.

In Step (c), a second (i.e. different) AAV *cap* gene is expressed (compared to Step (a) together with an AAV *rep* gene and viral helper genes in order to facilitate the replication of the AAV genome and to produce second recombinant AAV particles which are encapsidated by second AAV capsid polypeptides, thus producing recombinant AAV particles with a tropism towards the third host cells.

The second AAV *cap* gene is expressed in the second host cells. The second AAV cap gene is different from the first AAV cap gene. The second AAV *cap* gene is as defined above. The first AAV cap gene is not expressed in the second host cells.

The nucleic acid molecule encoding an AAV *rep* gene is as defined above. The nucleic acid molecules encoding an AAV rep gene used in Steps (a) and (c) may be the same or different.

The nucleic acid molecules encoding the viral helper genes are as defined above. The nucleic acid molecules encoding the viral helper genes used in Steps (a) and (c) may be the same or different.

Step (d) comprises culturing the second host cells in a culture medium under conditions such that second recombinant AAV particles comprising the recombinant AAV genome are produced, the second recombinant AAV particles each being encapsidated by a capsid comprising the second capsid polypeptides. Conditions for the culturing of host cells for the production of AAVs are well known in the art. During the culturing step, Rep polypeptides and second Cap polypeptides will be produced, and also viral helper polypeptides. No first Cap polypeptides will be produced (because the second host cells do not express first AAV *cap* genes). The recombinant AAV genome will be replicated and encapsidated by the second Cap (capsid) polypeptides, thus producing second recombinant AAV particles.

Step (e) is optional. It comprises purifying and/or isolating second recombinant AAV particles from the second host cells or from the culture medium. Methods of purifying and/or isolating recombinant AAV particles from host cells and the culture media are well known in the art.

The second AAV capsid polypeptides confer a tropism on the second recombinant AAV particles towards third host cells (target cells). Preferably, the third host cells are ones which do not have the same AAV receptors as high efficiency producer/manufacturing cell lines (e.g. HEK293, PerC6, 911). Preferably, the first AAV capsid polypeptides do not confer an efficient tropism on AAV particles which are encapsidated by such polypeptides towards the third host cells. The second recombinant AAV particles will have a stronger tropism towards the third host cells than towards the first or second host cells. Preferably, the third host cells are not of the same type as either the first or second host cells.

Preferred third host cells (target cells) include but are not limited to neurons (preferably retinal neurons), hepatocytes, muscle cells, stem cells (e.g. haematopoietic stem cells, mesenchymal stem cells, embryonic stem cells, adipose stem cells, and induced pluripotent stem cells and their derivatives), immune cells (including B and T lymphocytes, natural killer cells, monocytes and macrophages and granulocytes), endothelial cells, cardiovascular cells, epithelial cells, mesenchymal cells, pancreatic a cells and pancreatic b cells, cardiomyocytes, spleen cells, fat cells, glial cells, fibroblasts, Kupffer cells and cancer cells (e.g. leukaemia, lymphoma, myeloma, carcinoma, sarcoma, melanoma cells).

In some embodiments, the process additionally comprises Step (f): infecting a third host cell with second recombinant AAV particles of the invention. The second recombinant AAV particles will be encapsidated by second capsid polypeptides, thus giving the second recombinant AAV particles a tropism towards the third host cells. This facilitates the infection of the third host cells by the second recombinant AAV particles.

Preferably, the process steps are carried out in the order specified.

Also disclosed is a process for pseudo-typing recombinant AAV particles to alter their host cell tropism range, the process comprising Steps (a)-(d) of the invention, and also optionally Step (e).

WO2019/020992 discloses that transcription of the Late adenoviral genes can be regulated (e.g. inhibited) by the insertion of a repressor element into the Major Late Promoter. By "switching off" expression of the adenoviral Late genes, the cell's protein-manufacturing capabilities can be diverted toward the production of a desired recombinant protein or recombinant AAV particles.

In some embodiments, therefore, the recombinant adenovirus (i.e. adenoviral vector) comprises a repressible Major Late Promoter (MLP) and a plurality of adenoviral late genes, wherein the MLP comprises one or more repressor elements which are capable of regulating or controlling transcription of the adenoviral late genes, and wherein one or more of the repressor elements are inserted downstream of the MLP TATA box.

In other embodiments, the recombinant adenovirus (i.e. adenoviral vector) comprises (a) a plurality of adenoviral early genes, and (b) a plurality of adenoviral late genes under the control of a Major Late Promoter (MLP), and (c) a transgene (e.g. comprising AAV *rep* and *cap* genes), wherein the MLP comprises one or more repressor elements which are capable of regulating or controlling transcription of the adenoviral late genes, and wherein one or more of the repressor elements are inserted downstream of the MLP TATA box.

Preferred features for producing viral (preferably AAV) particles include the following:
- wherein the one or more repressor elements are inserted between the MLP TATA box and the +1 position of transcription.
- wherein the repressor element is one which is capable of being bound by a repressor protein.
- wherein a gene encoding a repressor protein which is capable of binding to the repressor element is encoded within the adenoviral genome.
- wherein the repressor protein is transcribed under the control of the MLP.
- wherein the repressor protein is the tetracycline repressor, the lactose repressor or the ecdysone repressor, preferably the tetracycline repressor (TetR).
- wherein the repressor element is a tetracycline repressor binding site comprising or consisting of the sequence set forth in SEQ ID NO: 28.
- wherein the nucleotide sequence of the MLP comprises or consists of the sequence set forth in SEQ ID NO: 29 or 30.
- wherein the presence of the repressor element does not affect production of the adenoviral E2B protein.
- wherein the adenoviral vector encodes the adenovirus L4 100K protein and wherein the L4 100K protein is not under control of the MLP.
- wherein a transgene is inserted within one of the adenoviral early regions, preferably within the adenoviral E1 region instead of in a Transfer Plasmid.
- wherein the transgene comprises a Tripartite Leader (TPL) in its 5'-UTR.
- wherein the transgene encodes a therapeutic polypeptide.
- wherein the transgene encodes a virus protein, preferably a protein that is capable of assembly in or outside of a cell to produce a virus-like particle, preferably wherein the transgene encodes Norovirus VP1 or Hepatitis B HBsAG.

Preferably, one or more of the repressor elements are inserted downstream of the MLP TATA box. Preferably, the transgene comprises AAV *rep* and *cap* genes.

There are many established algorithms available to align two amino acid or nucleic acid sequences. Typically, one sequence acts as a reference sequence, to which test sequences may be compared. The sequence comparison algorithm calculates the percentage sequence identity for the test sequence(s) relative to the reference sequence, based on the designated program parameters. Alignment of amino acid or nucleic acid sequences for comparison may be conducted, for example, by computer-implemented algorithms (e.g. GAP, BESTFIT, FASTA or TFASTA), or BLAST and BLAST 2.0 algorithms.

Percentage amino acid sequence identities and nucleotide sequence identities may be obtained using the BLAST methods of alignment (Altschul et al. (1997), "Gapped BLAST and PSI-BLAST: a new generation of protein database search programs", Nucleic Acids Res. 25:3389-3402; and http://www.ncbi.nlm.nih.gov/BLAST). Preferably the standard or default alignment parameters are used.

Standard protein-protein BLAST (blastp) may be used for finding similar sequences in protein databases. Like other BLAST programs, blastp is designed to find local regions of similarity. When sequence similarity spans the whole sequence, blastp will also report a global alignment, which is the preferred result for protein identification purposes. Preferably the standard or default alignment parameters are used. In some instances, the "low complexity filter" may be taken off.

BLAST protein searches may also be performed with the BLASTX program, score=50, wordlength=3. To obtain gapped alignments for comparison purposes, Gapped BLAST (in BLAST 2.0) can be utilized as described in Altschul et al. (1997) Nucleic Acids Res. 25: 3389. Alternatively, PSI-BLAST (in BLAST 2.0) can be used to perform an iterated search that detects distant relationships between molecules. (See Altschul et al. (1997) supra). When utilizing BLAST, Gapped BLAST or PSI-BLAST, the default parameters of the respective programs may be used.

With regard to nucleotide sequence comparisons, MEGABLAST, discontiguous-megablast, and blastn may be used to accomplish this goal. Preferably the standard or default alignment parameters are used. MEGABLAST is specifically designed to efficiently find long alignments between very similar sequences. Discontiguous MEGABLAST may be used to find nucleotide sequences which are similar, but not identical, to the nucleic acids of the invention.

The BLAST nucleotide algorithm finds similar sequences by breaking the query into short subsequences called words. The program identifies the exact matches to the query words first (word hits). The BLAST program then extends these word hits in multiple steps to generate the final gapped alignments. In some embodiments, the BLAST nucleotide searches can be performed with the BLASTN program, score=100, wordlength=12.

One of the important parameters governing the sensitivity of BLAST searches is the word size. The most important reason that blastn is more sensitive than MEGABLAST is that it uses a shorter default word size (11). Because of this, blastn is better than MEGABLAST at finding alignments to related nucleotide sequences from other organisms. The word size is adjustable in blastn and can be reduced from the default value to a minimum of 7 to increase search sensitivity.

A more sensitive search can be achieved by using the newly-introduced discontiguous megablast page (www.ncbi.nim.nih.gov/Web/Newsitr/FallWinter02/blastlab.html). This page uses an algorithm which is similar to that reported by Ma et al. (Bioinformatics. 2002 Mar; 18(3): 440-5). Rather than requiring exact word matches as seeds for alignment extension, discontiguous megablast uses non-contiguous word within a longer window of template. In coding mode, the third base wobbling is taken into consideration by focusing on finding matches at the first and second codon positions while ignoring the mismatches in the third position. Searching in discontiguous MEGABLAST using the same word size is more sensitive and efficient than standard blastn using the same word size. Parameters unique for discontiguous megablast are: word size: 11 or 12; template: 16, 18, or 21; template type: coding (0), non-coding (1), or both (2).

In some embodiments, the BLASTP 2.5.0+ algorithm may be used (such as that available from the NCBI) using the default parameters.

In other embodiments, a BLAST Global Alignment program may be used (such as that available from the NCBI) using a Needleman-Wunsch alignment of two protein sequences with the gap costs: Existence 11 and Extension 1.

The nucleic acid molecules, plasmids and vectors of the invention may be made by any suitable technique. Recombinant methods for the production of the nucleic acid molecules and production cell lines of the invention are well known in the art (e.g. "Molecular Cloning: A Laboratory Manual" (Fourth Edition), Green, MR and Sambrook, J., (updated 2014)).

### BRIEF DESCRIPTION OF THE FIGURES

Figure 1 shows production of recombinant AAV2 vectors in HEK293 cells via co-infection of AAV2, AAV5, AAV6 or AAV9 with Tetracycline-enabled repression adenovirus encoding AAV Rep and Cap2 genes (TERA-RepCap2).
Figure 2 shows production of recombinant AAV9 vectors in HEK293 cells via co-infection of either recombinant AAV2 or recombinant AAV9 with a tetracycline-enabled repression adenovirus encoding AAV Rep and Cap9 genes (TERA-RepCap9).

### EXAMPLES

The present invention is further illustrated by the following Examples, in which parts and percentages are by weight and degrees are Celsius, unless otherwise stated. It should be understood that these Examples, while indicating preferred embodiments of the invention, are given by way of illustration only.

### General methods

### Recovery of adenoviral vectors containing AAV components

All recombinant adenoviral vectors have been initially recovered in HEK293 cells from plasmid DNA encoding each viral genome. Plasmids (20 µg) were linearised with Swal restriction enzymes to release virus ITRs from the bacterial plasmid backbone and purified using genomic Purelink DNA extraction kit (Invitrogen, CA, USA). HEK293 cells were seeded in T25 tissue culture flasks, at a density of 2×10⁶ cells per flask, for 24-hours before transfection. Each flask was transfected with 2.5 µg of linearised DNA using Lipofectamine 2000 (Invitrogen) according to the manufacturer's protocol. Transfection media were exchanged with fresh DMEM containing 2% FBS (supplemented with doxycycline 0.5 µg/mL or DMSO) after 4 hours. Recombinant viruses were harvested from growth media ~12 days post-transfection upon observation of full CPE. Single clone was isolated by two-rounds of serial dilution and further propagated in HEK293 cells (and cultured with doxycycline 0.5 µg/mL or DMSO). Viruses were harvested by three rounds of freeze-thaw at day 3 post-infection. Cellular debris were pelleted by centrifugation and supernatant passed through a 0.2 µm filter. For large scale virus amplification and purification, HEK293 cells were seeded in Corning^{®} HYPERFlask^{®} (Sigma-Aldrich, MO, USA) for 48-hours so that they are ~95% confluent before infection. For adenoviral vectors containing rep and cap genes, viral stock was used to infect each HYPERFlask at an MOI of 3 for 3 days. For adenoviral vectors containing a modified major late promoter that is regulated by the tetR protein, cell cultures were supplemented with doxycycline 0.5 µg/mL. Cells are harvested upon display of full CPE and virus released by three rounds of freeze-thaw. Virus were purified by two rounds of CsCl gradient banding, with Benzonase 250 U/mL (Sigma-Aldrich, MO, USA) added after the first round to degrade free DNA.

### AAV transduction assay

Transduction-competent AAV can be measured using a modified TCID50 assay. HEK293 cells can be seeded in 96-well tissue culture plates at a density of 1×10⁴ cells per well for 24-hours. Eight 10-fold serial dilutions of each AAV crude lysate stock can then be made in DMEM containing 2% FBS at a total volume of 1.2mL. Ten replicates of each diluted sample (1×10⁻² to 1×10⁻¹⁰) can then be added at a volume of 100uL per well on each plate. 100uL of DMEM containing 2% FBS can be added to the final two columns as a negative control. Plates can then be observed for the presence of EGFP expressing cells from each well 96hpi using a fluorescent microscope (EVOS FL imaging system, ThermoFisher Scientific, MA, USA). Transducing units per mL can be calculated using KÄRBER-SPEARMAN statistical method.

### Quantification of viral genomes and gene expression using qPCR

For quantification of total adenovirus genomes in HEK293 cells, total DNA was extracted from culture media and cellular lysates using Purelink genomic DNA miniprep kit (Invitrogen, CA, USA). Five microlitres of DNA eluent were used in qPCR reactions using TaqMan Fast Advanced Master Mix (Applied Biosystems, CA, USA) in a StepOnePlus Real-Time PCR System (Applied Biosystems, CA, USA).

For quantification of genome encapsulated AAV and adenovirus particles, 2 µL of viral samples, harvested from culture medium or cell lysates, were treated with 1U of TURBO DNase (ThermoFisher Scientific, MA, USA) in a 20 µL reaction for 2-hours at 37°C. TURBO DNase was heat-inactivated at 75°C for 10-minutes. Five microlitres of samples diluted at 1:200 using nuclease-free water were used in the PCR reaction to quantify encapsulated Ad5 using Ad5 hexon primers and probe, while EGFP primers and probe were used to quantify encapsulated AAV genomes. Titres of total AAV vectors produced using helper adenovirus were determined by subtraction of genome encapsulated adenoviruses. Standard curves for qPCR analyses were generated using a gBLOCK gene fragment suspended in nuclease-free water (Integrated DNA Technologies, IA, USA) and CT values of PCR reaction was used to calculate DNA copy number by extrapolation to the standard curves (a qPCR standard of 3×10⁸-3×10¹ copies/well).

### AAV capsid quantification by ELISA

Detection of assembled AAV2 capsids can be carried out using AAV2 titration ELISA kit (Progen, Heidelberg, Germany) according to the manufacturer's instruction.

### Example 1: Production of AAV in first host cells via either plasmid or adenoviral approaches

Helper-free AAV2 and AAV9 production was carried out according to Takara AAVpro Helper Free System (Clontech, CA, USA). For AAV2 and AAV9 production in 6-well tissue culture treated plates, HEK293 cells were seeded at a density of 7.5×10⁴ cells per well for 24 hours prior to transfection. For helper-free production, each well was transfected with 2.5 µg of plasmid DNA, containing pHelper, pAAV-CMV-EGFP and pRepCap2 or pRepCap9 diluted with Opti-MEM (Gibco, MA, USA) at a DNA mass ratio of 1:1:1, and complexed using linear PEI 25kDA (Polysciences) at a 1:3 DNA to PEI mass ratio.

For AAV production using adenoviral vectors containing AAV rep and cap genes, HEK293 cells were seeded in 48-well tissue culture plates, at a density of 7.5×10⁴ cells per well, for 24-hours prior to production. Each well was co-infected with AAV containing an AAV genome encoding EGFP and an adenovirus containing both the rep and cap genes. Infection media were exchanged with fresh DMEM containing 2% FBS (supplemented with doxycycline 0.5 µg/mL or DMSO) 4 hours post treatment. To determine efficiency of the DNase reaction in degrading free-DNA, a helper-free production control was included with each experiment, wherein the stuffer plasmid pUC19 was used in place of pRepCap2 or pRepCap9. AAV vectors were harvested via three rounds of freeze-thaw of cells suspended in growth media. Cells were pelleted by centrifugation at 3000g for 20 minutes and supernatant passed through a 0.2 µm filter.

### Example 2: Infection of second host cells using different AAV serotypes to produce AAV2

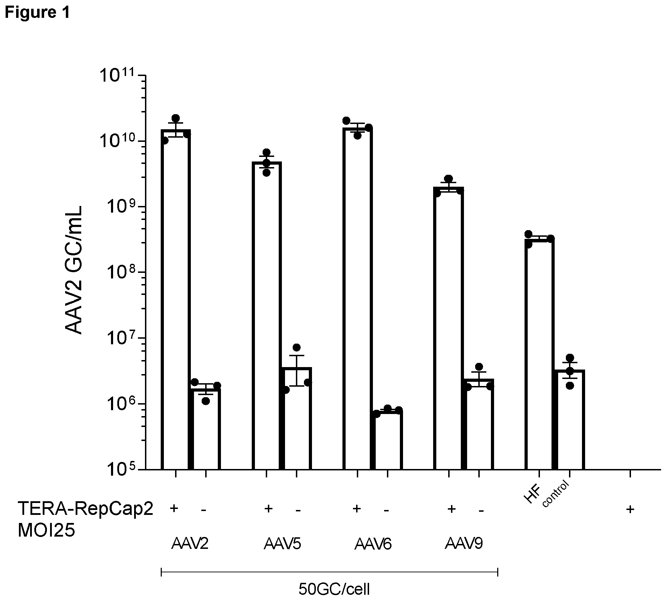

Figure 1 shows production of recombinant AAV2 vectors in HEK293 cells via co-infection of AAV2, AAV5, AAV6 or AAV9 with Tetracycline-enabled repression adenovirus encoding AAV Rep and Cap2 genes (TERA-RepCap2). The data shows that using different serotypes of AAV at the same concentration can significantly impact the amount of AAV that is subsequently produced from the second host cells. AAV2 and AAV6 are known to infect HEK293 cells efficiently, whilst AAV9 and AAV5 are known to infect HEK293 cells less efficiently. This infection efficiency directly impacts output yield for each serotype. These production levels are then directly compared to the helper-free plasmid transfection method.

In this example, HEK293 cells were seeded in 48-well tissue culture plate format at 9e4 cells/well for 24 hours. HEK293 cells were triple transfected with the helper-free plasmids or co-infected using a tetracycline-enabled repression adenovirus virus encoding AAV Rep and Cap2 with recombinant AAV2, AAV5, AAV6, or AAV9 encoding the EGFP reporter at 50 genome copies per cell.

AAV vectors were harvested 96 hours post-transduction and encapsulated AAV particles quantified by QPCR.

### Example 3: Infection of second host cells using AAV2 and AAV9 to produce AAV9

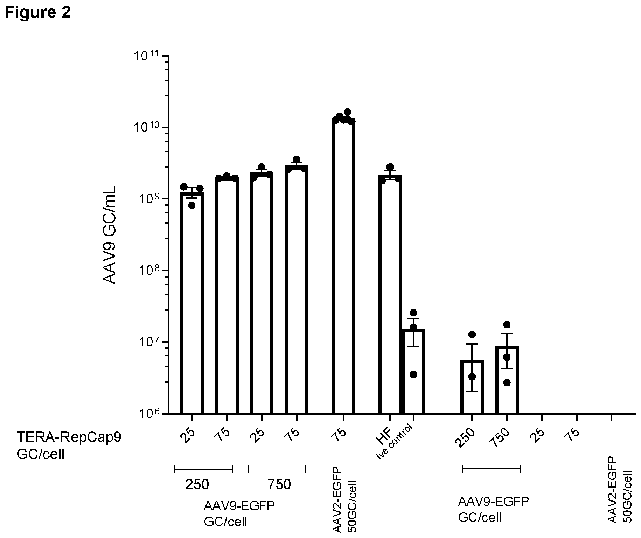

Figure 2 shows production of recombinant AAV9 vectors in HEK293 cells via co-infection of either recombinant AAV2 or recombinant AAV9 with a tetracycline-enabled repression adenovirus encoding AAV Rep and Cap9 genes (TERA-RepCap9). The production of recombinant AAV is compared to helper-free plasmid transfection method. The data shows that the infection of HEK293 cells with AAV2 at an MOI of 50 and TERA-RepCap9 at an MOI of 75 produces 4.69-fold more AAV9 than if AAV9 at an MOI at 750 and TERA-RepCap9 at an MOI of 75 is used. Therefore, per AAV molecule, using AAV2 to make AAV9 is approximately 70-fold more efficient that using AAV9 to make AAV9 in HEK293 cells. This represents a fundamental exemplification of the invention.

In this example, HEK293 cells were seeded in 48-well tissue culture plate format at 9e4 cells/well for 24 hours. HEK293 cells were triple transfected with the helper-free plasmids or co-infected at the indicated MOI using TERA-RepCap9 virus encoding AAV Rep and Cap9 with recombinant AAV2 or AAV9 vectors encoding the EGFP reporter. AAV vectors were harvested 96 hours post-transduction and encapsulated AAV particles quantified by QPCR.

### Example 4: Infection of target cells

Target cells are transduced with AAV from a second host cell. This is achieved through a range of approaches that are suitable for the disease condition being treated. Delivery to third host cells is achieved via direct *in vivo* delivery by needle injection to a patient. This is directly into a therapeutic target tissue, or delivery intravenously where the second capsid polypeptides enable infection of the target cells.

Cells can also be infected *ex vivo* by the application of AAV to cells that have been isolated from a patient. These infected cells are re-administered to a patient.

### SEQUENCE LISTING FREE TEXT

<210> 1 <223> AAV1 - Capsid Nucleotide Sequence (AF063497.1)
<210> 2 <223> AAV1 - Capsid Protein Sequence
<210> 9 <223> AAV5 - Capsid Nucleotide Sequence (AF085716.1)
<210> 10 <223> AAV5 - Capsid Protein Sequence
<210> 11 <223> AAV6 - Capsid Nucleotide Sequence (AF028704.1)
<210> 12 <223> AAV6 - Capsid Protein Sequence
<210> 13 <223> AAV7 - Capsid Nucleotide Sequence (AF513851.1)
<210> 14 <223> AAV7 - Capsid Protein Sequence
<210> 15 <223> AAV8 - Capsid Nucleotide Sequence (AF513852.1)
<210> 16 <223> AAV8 - Capsid Protein Sequence
<210> 17 <223> AAV9 - Capsid Nucleotide Sequence (AY530556.1)
<210> 18 <223> AAV9 - Capsid Protein Sequence
<210> 28 <223> TetR binding site
<210> 29 <223> Modified MLP
<210> 30 <223> Modified MLP

## Claims

1. A process for producing recombinant adeno-associated virus (AAV) particles, the process comprising the steps:
(a) culturing a population of first host cells, wherein each first host cell comprises:
(i) a nucleic acid molecule encoding a recombinant AAV genome;
(ii) a nucleic acid molecule encoding a first AAV cap gene which encodes first AAV capsid polypeptides, wherein the first AAV capsid polypeptides confer a tropism on AAV particles which comprise such polypeptides towards second host cells;
(iii) a nucleic acid molecule encoding an AAV rep gene;
(iv) nucleic acid molecules encoding viral helper genes;
such that the expression of each of (i)-(iv) in the first host cells is sufficient to produce first recombinant AAV particles comprising the AAV genome in the first host cells, wherein the first AAV particles are encapsidated by first AAV capsid polypeptides;
(b) infecting a population of second host cells with the first recombinant AAV particles which are produced from Step (a);
(c) expressing in the population of second host cells:
(i) a nucleic acid molecule encoding a second AAV cap gene which encodes second AAV capsid polypeptides, wherein the second AAV capsid polypeptides confer a tropism on AAV particles which comprise such polypeptides towards third host cells;
(ii) a nucleic acid molecule encoding an AAV rep gene;
(iii) nucleic acid molecules encoding viral helper genes;
wherein all of (i)-(iii) are independently either present in the second host cells or are subsequently introduced into the second host cells,
such that the expression of each of (i)-(iii) in the second cells is sufficient to produce second recombinant AAV particles comprising the recombinant AAV genome in the second host cells, wherein the second recombinant AAV particles are encapsidated by the second AAV capsid polypeptides;
(d) culturing the second host cells in a culture medium under conditions such that second recombinant AAV particles comprising the recombinant AAV genome are produced, the second recombinant AAV particles each being encapsidated by a capsid comprising the second capsid polypeptides;
and optionally,
(e) purifying and/or isolating second recombinant AAV particles from the second host cells or from the culture medium.

2. The process as claimed in claim 1, wherein Step (a) additionally comprises the prior step of introducing the nucleic acid molecule encoding a recombinant AAV genome into the first host cells.

3. The process as claimed in claim 1 or claim 2, wherein the first host cells and/or second host cells are HEK293, HEK293T, HEK293A, PerC6 or 911 cells.

4. The process as claimed in any one of the preceding claims, wherein the nucleic acid molecule encoding the recombinant AAV genome introduced into the first host cells is in the form of a plasmid, recombinant adenovirus, or is integrated into the genome of a cell or a recombinant AAV particle.

5. The process as claimed in any one of the preceding claims, wherein the nucleic acid molecule encoding the first AAV cap gene and/or the nucleic acid encoding the *rep* gene is introduced into the first host cells in the form of a plasmid or in a recombinant adenovirus genome.

6. The process as claimed in any one of the preceding claims, wherein the nucleic acid molecule encoding the second AAV cap gene and/or the nucleic acid encoding the rep gene is introduced into the second host cells in the form of a plasmid or in a recombinant adenovirus genome.

7. The process as claimed in any one of the preceding claims, wherein the amino acid sequence identity between the first and second AAV capsid polypeptides is less than 99.5%, preferably less than 99%, 98%, 97%, 96%, 95% or less than 90%; and at least 50%.

8. The process as claimed in any one of the preceding claims, wherein the first recombinant AAV particles are of serotype 2 (AAV2).

9. The process as claimed in any one of the preceding claims, wherein the second recombinant AAV particles are of serotype 9 (AAV9), or modified or mutated derivative of serotype 9.

10. The process as claimed in any one of the preceding claims, wherein the third host cells are selected from the group consisting of neurons (e.g. retinal neurons), hepatocytes, muscle cells, stem cells (preferably mesenchymal stem cells, haematopoietic stem cells, embryonic stem cells, adipose stem cells and induced pluripotent stem cells and their derivatives), immune cells (including B and T lymphocytes, natural killer cells, monocytes and macrophages and granulocytes), endothelial cells, cardiovascular cells, epithelial cells, mesenchymal cells, pancreatic a cells or b cells, cardiomyocytes, spleen cells, fat cells, glial cells, fibroblasts, Kupffer cells and cancer cells (preferably leukaemia, lymphoma, myeloma, carcinoma, sarcoma or melanoma cells).

11. The process as claimed in any one of the preceding claims, wherein the nucleic acid molecules encoding the first and/or second *cap* genes and/or the *rep* genes are present in a recombinant adenovirus (AV), wherein the recombinant adenovirus comprises a repressible Major Late Promoter (MLP) and a plurality of adenoviral late genes, wherein the MLP comprises one or more repressor elements which are capable of regulating or controlling transcription of the adenoviral late genes, and wherein one or more of the repressor elements are inserted downstream of the MLP TATA box.

## Patentansprüche

1. Prozess zur Herstellung rekombinanter Adeno-assoziierter Virenpartikel (AAV), wobei der Prozess die Schritte umfasst:
a) Kultivieren einer Population erster Wirtszellen, wobei jede erste Wirtszelle umfasst:
(i) ein Nukleinsäuremolekül, das ein rekombinantes AAV-Genom codiert;
(ii) ein Nukleinsäuremolekül, das ein erstes AAV-*Cap*-Gen codiert, das erste AAV-Capsidpolypeptide codiert, wobei die ersten AAV-Capsidpolypeptide AAV-Partikeln, die solche Polypeptide umfassen, einen Tropismus gegenüber zweiten Wirtszellen verleihen;
(iii) ein Nukleinsäuremolekül, das ein AAV*Rep*-Gen codiert;
(iv) Nukleinsäuremoleküle, die virale Helfergene codieren;
sodass die Expression von jedem von (i)-(iv) in den ersten Wirtszellen ausreicht, um erste rekombinante AAV-Partikel, die das AAV-Genom umfassen, in den ersten Wirtszellen herzustellen, wobei die ersten AAV-Partikel von den ersten AAV-Kapsidpolypeptiden eingekapselt sind;
b) Infizieren einer Population zweiter Wirtszellen mit den ersten rekombinanten AAV-Partikeln, die in Schritt (a) hergestellt werden;
(c) Exprimieren in der Population zweiter Wirtszellen:
(i) ein Nukleinsäuremolekül, das ein zweites AAV- *Cap* -Gen codiert, das zweite AAV-Capsidpolypeptide codiert, wobei die zweiten AAV-Capsidpolypeptide AAV-Partikeln, die solche Polypeptide umfassen, einen Tropismus gegenüber dritten Wirtszellen verleihen;
(ii) ein Nukleinsäuremolekül, das ein AAV*Rep*-Gen codiert;
(iii) Nukleinsäuremoleküle, die virale Helfergene codieren;
wobei alle von (i)-(iii) unabhängig voneinander entweder in den zweiten Wirtszellen vorhanden sind oder nachträglich in die zweiten Wirtszellen eingeführt werden,
sodass die Expression von jedem von (i)-(iii) in den zweiten Wirtszellen ausreicht, um zweite rekombinante AAV-Partikel, die das rekombinante AAV-Genom umfassen, in den zweiten Wirtszellen herzustellen, wobei die zweiten rekombinanten AAV-Partikel von den zweiten AAV-Kapsidpolypeptiden eingekapselt sind;
d) Kultivieren der zweiten Wirtszellen in einem Kulturmedium unter Bedingungen, sodass zweite rekombinante AAV-Partikel, die das rekombinante AAV-Genom umfassen, hergestellt werden, wobei die zweiten rekombinanten AAV-Partikel jeweils von einem Kapsid eingekapselt sind, das die zweiten Kapsidpolypeptide umfasst;
und optional,
e) Reinigen und/oder Isolieren zweiter rekombinanter AAV-Partikel aus den zweiten Wirtszellen oder aus dem Kulturmedium.

2. Prozess nach Anspruch 1, wobei Schritt (a) zusätzlich den vorherigen Schritt des Einführens des Nukleinsäuremoleküls, das ein rekombinantes AAV-Genom codiert, in die ersten Wirtszellen umfasst.

3. Prozess nach Anspruch 1 oder Anspruch 2, wobei die ersten Wirtszellen und/oder zweiten Wirtszellen HEK293-, HEK293T-, HEK293A-, PerC6- oder 911-Zellen sind.

4. Prozess nach einem der vorstehenden Ansprüche, wobei das in die ersten Wirtszellen eingeführte Nukleinsäuremolekül, welches das rekombinante AAV-Genom codiert, in der Form eines Plasmids, eines rekombinanten Adenovirus ist, oder in das Genom einer Zelle oder eines rekombinanten AAV-Partikels integriert ist.

5. Prozess nach einem der vorstehenden Ansprüche, wobei das Nukleinsäuremolekül, welches das erste AAV-*Cap*-Gen codiert und/oder die Nukleinsäure, die das *Rep*-Gen cordiert, in der Form eines Plasmids oder in einem rekombinanten Adenovirusgenom in die ersten Wirtszellen eingeführt wird.

6. Prozess nach einem der vorstehenden Ansprüche, wobei das Nukleinsäuremolekül, welches das zweite AAV-*Cap*-Gen codiert und/oder die Nukleinsäure, die das Rep-Gen codiert, in der Form eines Plasmids oder in einem rekombinanten Adenovirusgenom in die zweiten Wirtszellen eingeführt wird.

7. Prozess nach einem der vorstehenden Ansprüche, wobei die Aminosäuresequenzidentität zwischen dem ersten und zweiten AAV-Capsidpolypeptid kleiner als 99,5%, vorzugsweise kleiner als 99%, 98%, 97%, 96%, 95 % oder kleiner als 90% ist; und zumindest 50% beträgt.

8. Prozess nach einem der vorstehenden Ansprüche, wobei die ersten rekombinanten AAV-Partikel vom Serotyp 2 (AAV2) sind.

9. Prozess nach einem der vorstehenden Ansprüche, wobei die zweiten rekombinanten AAV-Partikel vom Serotyp 9 (AAV9), oder einem modifizierten oder mutierten Derivat des Serotyps 9 sind.

10. Prozess nach einem der vorstehenden Ansprüche, wobei die dritten Wirtszellen aus der Gruppe ausgewählt sind, bestehend aus Neuronen (z. B. retinalen Neuronen), Hepatozyten, Muskelzellen, Stammzellen (vorzugsweise mesenchymalen Stammzellen, hämatopoetischen Stammzellen, embryonalen Stammzellen, Fettgewebestammzellen und induzierten pluripotenten Stammzellen und deren Derivaten), Immunzellen (B- und T-Lymphozyten, natürliche Killerzellen, Monozyten und Makrophagen und Granulozyten beinhaltend), Endothelzellen, kardiovaskulären Zellen, Epithelzellen, Mesenchymzellen, Pankreas-A-Zellen oder Pankreas-B-Zellen, Kardiomyozyten, Milzzellen, Fettzellen, Gliazellen, Fibroblasten, Kupffer'schen Zellen und Krebszellen (vorzugsweise Leukämie-, Lymphom-, Myelom-, Karzinom-, Sarkom- oder Melanomzellen).

11. Prozess nach einem der vorstehenden Ansprüche, wobei die Nukleinsäuremoleküle, die das erste und/oder zweite Cap-Gen und/oder die Rep-Gene codieren, in einem rekombinanten Adenovirus (AV) vorhanden sind, wobei das rekombinante Adenovirus einen unterdrückbaren Major Late Promoter (MLP) und eine Vielzahl von späten Adenovirusgenen umfasst, wobei der MLP ein oder mehrere Repressorelemente umfasst, die in der Lage sind, Transkription der späten Adenovirusgene zu regulieren oder zu kontrollieren, und wobei ein oder mehrere der Repressorelemente stromabwärts der MLP-TATA-Box eingefügt sind.

## Revendications

1. Processus de production de particules de virus adéno-associé (AAV) recombinantes, le processus comprenant les étapes suivantes :
a) la culture d'une population de premières cellules hôtes, dans lequel chaque première cellule hôte comprend :
(i) une molécule d'acide nucléique codant un génome AAV recombinant ;
(ii) une molécule d'acide nucléique codant un premier gène *cap* d'AAV qui code des premiers polypeptides de capside d'AAV, dans lequel les premiers polypeptides de capside d'AAV confèrent un tropisme aux particules d'AAV qui comprennent de tels polypeptides envers des deuxièmes cellules hôtes ;
(iii) une molécule d'acide nucléique codant un gène *rep* d'AAV ;
(iv) des molécules d'acide nucléique codant des gènes auxiliaires viraux ;
de telle sorte que l'expression de chacune de (i) à (iv) dans les premières cellules hôtes soit suffisante pour produire des premières particules d'AAV recombinantes comprenant le génome d'AAV dans les premières cellules hôtes, dans lequel les premières particules d'AAV sont encapsidées par des premiers polypeptides de capside d'AAV ;
b) l'infection d'une population de deuxièmes cellules hôtes avec les premières particules d'AAV recombinantes qui sont produites à l'étape (a) ;
(c) l'expression, dans la population de deuxièmes cellules hôtes, de ce qui suit :
(i) une molécule d'acide nucléique codant un second gène *de cap* d'AAV qui code des seconds polypeptides de capside d'AAV, dans lequel les seconds polypeptides de capside d'AAV confèrent un tropisme aux particules d'AAV qui comprennent de tels polypeptides envers des troisièmes cellules hôtes ;
(ii) une molécule d'acide nucléique codant un gène *rep* d'AAV ;
(iii) des molécules d'acide nucléique codant des gènes auxiliaires viraux ;
dans lequel toutes les (i) à (iii) sont indépendamment présentes dans les deuxièmes cellules hôtes ou sont ultérieurement introduites dans les deuxièmes cellules hôtes,
de telle sorte que l'expression de chacune de (i) à (iii) dans les deuxièmes cellules soit suffisante pour produire des secondes particules d'AAV recombinantes comprenant le génome d'AAV recombinant dans les deuxièmes cellules hôtes, dans lequel les secondes particules d'AAV recombinantes sont encapsidées par les seconds polypeptides de capside d'AAV ;
d) la culture des deuxièmes cellules hôtes dans un milieu de culture dans des conditions telles que des secondes particules d'AAV recombinantes comprenant le génome d'AAV recombinant soient produites, les secondes particules d'AAV recombinantes étant chacune encapsidées par une capside comprenant les seconds polypeptides de capside ;
et facultativement,
e) la purification et/ou l'isolement de secondes particules d'AAV recombinantes à partir des deuxièmes cellules hôtes ou du milieu de culture.

2. Processus selon la revendication 1, dans lequel l'étape (a) comprend en outre l'étape préalable consistant à introduire la molécule d'acide nucléique codant pour un génome AAV recombinant dans les premières cellules hôtes.

3. Processus selon la revendication 1 ou 2, dans lequel les premières cellules hôtes et/ou les deuxièmes cellules hôtes sont des cellules HEK293, HEK293T, HEK293A, PerC6 ou 911.

4. Processus selon l'une quelconque des revendications précédentes, dans lequel la molécule d'acide nucléique codant le génome AAV recombinant introduit dans les premières cellules hôtes se présente sous la forme d'un plasmide, d'un adénovirus recombinant, ou est intégrée dans le génome d'une cellule ou d'une particule AAV recombinante.

5. Processus selon l'une quelconque des revendications précédentes, dans lequel la molécule d'acide nucléique codant le premier gène *cap* d'AAV et/ou l'acide nucléique codant le gène *rep* est introduit dans les premières cellules hôtes sous la forme d'un plasmide ou dans un génome d'adénovirus recombinant.

6. Processus selon l'une quelconque des revendications précédentes, dans lequel la molécule d'acide nucléique codant le second gène *cap* d'AAV et/ou l'acide nucléique codant le gène *rep* est introduit dans les deuxièmes cellules hôtes sous la forme d'un plasmide ou dans un génome d'adénovirus recombinant.

7. Processus selon l'une quelconque des revendications précédentes, dans lequel l'identité de séquence d'acides aminés entre les premier et second polypeptides de capside d'AAV est inférieure à 99,5 %, de préférence moins de 99 %, 98 %, 97 %, 96 %, 95 % ou moins de 90 % ; et d'au moins 50 %.

8. Processus selon l'une quelconque des revendications précédentes, dans lequel les premières particules d'AAV recombinantes sont de sérotype 2 (AAV2).

9. Processus selon l'une quelconque des revendications précédentes, dans lequel les secondes particules d'AAV recombinantes sont de sérotype 9 (AAV9), ou un dérivé modifié ou muté du sérotype 9.

10. Processus selon l'une quelconque des revendications précédentes, dans lequel les troisièmes cellules hôtes sont choisies dans le groupe consistant en des neurones (de préférence des neurones rétiniens), des hépatocytes, des cellules musculaires, des cellules souches (de préférence des cellules souches mésenchymateuses, des cellules souches hématopoïétiques, des cellules souches embryonnaires, des cellules souches adipeuses et des cellules souches pluripotentes induites et leurs dérivés), des cellules immunitaires (incluant des lymphocytes B et T, des cellules tueuses naturelles, des monocytes, des macrophages et des granulocytes), des cellules endothéliales, des cellules cardiovasculaires, des cellules épithéliales, des cellules mésenchymateuses, des cellules B pancréatiques ou des cellules A pancréatiques, des cardiomyocytes, des cellules de la rate, des cellules adipeuses, des cellules gliales, des fibroblastes, des cellules de Kupffer et des cellules cancéreuses (de préférence des cellules de leucémie, de lymphome, de myélome, de carcinome, de sarcome ou de mélanome).

11. Processus selon l'une quelconque des revendications précédentes, dans lequel les molécules d'acide nucléique codant les premier et/ou second gènes cap et/ou les gènes rep sont présentes dans un adénovirus recombinant (AV), dans lequel l'adénovirus recombinant comprend un promoteur tardif majeur (MLP) répressible et une pluralité de gènes tardifs adénoviraux, dans lequel le MLP comprend un ou plusieurs éléments répresseurs qui sont aptes à réguler ou à commander la transcription des gènes tardifs adénoviraux, et dans lequel un ou plusieurs des éléments répresseurs sont insérés en aval de la boîte TATA du MLP.
